# EUROPEAN PATENT APPLICATION

(11) **EP 1 895 307 A1**
(43) Date of publication of application: **05.03.2008**
(21) Application number: 07016194.8
(22) Date of filing: 17.08.2007
(51) Int. Cl.: G01N 35/00

(54) **Automated analyzer**

(30) Priority: 31.08.2006 JP 2006234831
(71) Applicant: Hitachi High-Technologies Corporation, Tokyo 105-8717 (JP)
(72) Inventor: Nakamura, Kazuhiro, Hitachinaka-shi Ibaraki 312-8504 (JP); Akutsu, Masashi, Hitachinaka-shi Ibaraki 312-8504 (JP)
(74) Representative: Strehl Schübel-Hopf & Partner

(57) **Abstract**

The present invention provides an automated analyzer not requiring the operator to set a new quality control material and to request measurement during the analysis of a sample, capable of continually verifying the appropriateness of the results of measurement of a standard sample by successively carrying out the measurement of a standard sample and that of a quality control material for the same reagent, and capable of guaranteeing the reliability of results of analysis with high reliability

A set standard sample is identified, and a quality control material relevant to a reagent requested by the standard sample and stored in a buffer is fed automatically to an analyzer module. The measurement of the quality control material is performed immediately after the measurement of the standard sample for the reagent. A warning signal is generated to prompt the operator to replenish the quality control material when it is decided from the amount of the residual quality control material stored in the buffer that the quality control material is insufficient.

## Description

### BACKGROUND OF THE INVENTION

### 1. Field of the Invention

The present invention relates to an automated analyzer for the quantitative and qualitative analysis of biological samples, such as blood samples and urine samples and, for example, to an automated analyzer having a quality control function.

### 2. Description of the Related Art

An automated analyzer needs to be calibrated by using a standard sample having a known concentration to ensure the measurement accuracy of the automated analyzer. For example, a biochemical automated analyzer, based on the principle of colorimetric method (colorimetric analyzing method) that makes an assay sample react with a reagent and measures a change in the color of the reagent according to the concentration of a specific component of the assay sample as an absorbance change, determines the concentration of a specific component of the assay sample by using a calibration curve representing the relation between absorbance and concentration. Also, the biochemical automated analyzer measures the standard sample periodically to correct the calibration curve and manages the quality control of results of analysis by periodically performing measurement using a quality control material (control) for ensuring accuracy.

An automated analyzer disclosed in JP-A 4-65676 loads a sample rack with assay samples, carries the sample rack from a rack receiving unit to an analyzing unit along a carrying line, and recovers the rack to a rack recovering unit after dispensation, wherein the automated analyzer stores a standard solution and a quality control material in a storage unit, and ejects the standard solution and the quality control material automatically from the storage unit for analysis after a predetermined number of assay samples has been analyzed or after a predetermined time has elapsed. This known automated analyzer can autonomously achieve quality control even if the operator does not request the analysis of the quality control material.

### SUMMARY OF THE INVENTION

The measurement of the standard sample and the quality control material is needed not only for the periodical control of the accuracy of analysis, but also at time after the passage of a predetermined time since the unsealing of a reagent container, when reagents are changed and when a measurement of the reagent measured for quality control exceeds a predetermined value, from a standpoint of the accuracy control of the result of analysis.

The invention mentioned in JP-A-4-65676 relates to a method of periodically controlling analysis accuracy, but does not consider anything about quality control when a standard sample necessary for a specific reagent needs to be measured and anything about a measuring method for occasions when the stored quality control materials are insufficient. When the standard samples are stored in the storage unit, a special arrangement is necessary to prevent the deterioration of the standard samples.

A conventional system that requests the measurement of both the standard sample and the quality control material requires the operator to set the standard sample and the quality control material and to request the analysis of both the standard sample and the quality control material using a specific reagent. Therefore, it is possible that measurement is performed for the quality control material with a reagent for which measurement is not essential, the operator forgets setting the quality control material corresponding to the specific reagent and the measurement of the quality control material corresponding to the reagent for measuring the standard sample is not carried out. Since the analysis of assay samples is executed between the measurement of the standard sample and that of the quality control material, the appropriateness of the measurement of the standard sample cannot be verified, and there is the possibility that the accuracy of measurements of the assay samples cannot be guaranteed.

Accordingly, it is an object of the present invention to provide an automated analyzer not requiring the operator to set a new quality control material during analysis and to request measurement, capable of improving the convenience of quality control and the reliability of measured results by continuously carrying out the measurement of a standard sample and that of a quality control material.

The present invention provides an automated analyzer capable of automatically selecting a quality control material relevant to a reagent for testing a standard sample when the measurement of the standard sample for calibration is requested, of displaying at least the name of the reagent for the measurement of the quality control material and a necessary amount of the quality control material, of identifying a standard sample when the standard sample is supplied thereto, of executing the measurement of the selected quality control material immediately after the measurement of the identified standard sample, and of automatically carrying out the measurement of the quality control material using the reagent.

It is preferable that this automated analyzer includes an output device that displays the name of the quality control material as a warning when it is expected that the amount of the quality control material is insufficient to prompt an operator to set a new quality control material. The automated analyzer is capable of automatically selecting another quality control material other than the quality control material whose amount is expected to be insufficient. Preferably, the automated analyzer includes an input/output device for selecting either of an automatic measuring mode and a manual measuring mode for the quality control measurement of a reagent when a request for a standard sample and the selection of a quality control material relevant to the reagent are executed.

The automated analyzer of the present invention automatically executes the measurement of a reagent using a quality control material at proper time subsequently to the measurement of the reagent using a standard sample without requiring request from the operator. Therefore, the appropriateness of the measurement of the standard sample can be verified, and highly reliable measurements can be guaranteed. Moreover, the insufficiency of the sample can be determined before analysis from the amount of the quality control material used for the automatic measurement of the quality control material and the operator can prepare and replenish the quality control material. Therefore, interruption or stoppage of measurement due to the insufficiency of the sample can be avoided and analysis can be continued.

### BRIEF DESCRIPTION OF THE DRAWINGS

Fig. 1 is a block diagram of an automated analyzer in an embodiment according to the present invention;
Fig. 2 is a perspective view of a biochemical analyzer module included in the automated analyzer shown in Fig. 1;
Fig. 3 is a flow chart of a quality control material measuring procedure to be executed by the automated analyzer shown in Fig. 1 when a standard sample is supplied to the automated analyzer;
Fig. 4 is a view of a display screen displaying information about quality control measurement for a reagent executed by the automated analyzer shown in Fig. 1; and
Fig. 5 is a view of a display screen displaying information about setting standard sample measurement for a reagent executed by the automated analyzer shown in Fig. 1.

### REFERENCE CHARACTERS

1 ... Sample rack feed unit, 2 ... ID Reader unit, 3 ... Carrying line, 4 ... Rack recirculating line, 5, 6, 7 and 8 ... Analyzer modules, 9 ... Sample rack storage unit, 10 ... Sample rack recovering unit, 11 .. Main control computer, 12, 13, 14, 15, 16 and 17 ... Computers, 18 ... Operating unit, 19 ... Display unit, 51, 61, 71 and 81 .. Siding lines, 91 ... Buffer unit

### DETAILED DESCRIPTION OF THE PREFERRED EMBODIMENTS

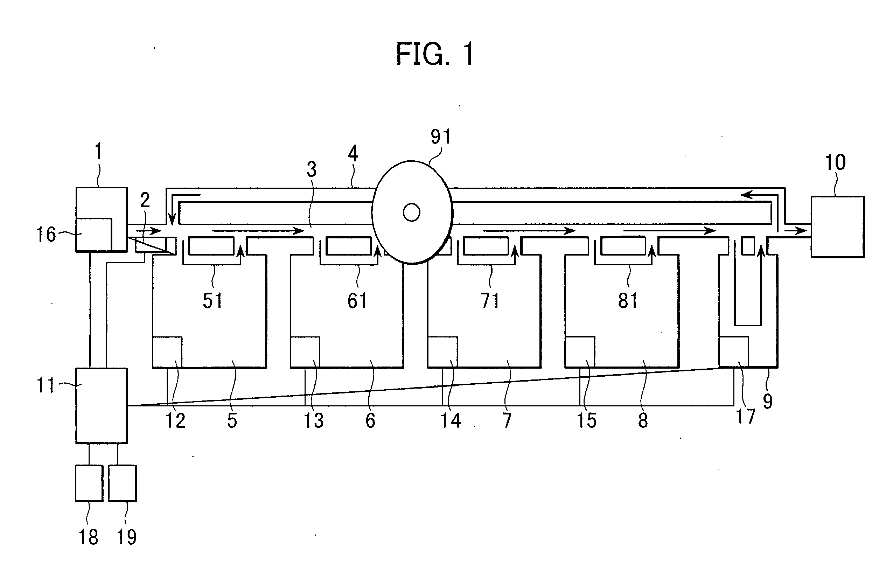

The constitution and operation of an automated analyzer in a preferred embodiment according to the present invention will be described with reference to Figs. 1 to 5.

Initially, the constitution of the automated analyzer in this embodiment will be described with reference to Fig. 1. Fig. 1 is a block diagram of an automated analyzer in this embodiment according to the present invention.

Referring to Fig. 1, the automated analyzer includes a sample rack feed unit 1, an ID reader unit 2, a rack carrying line 3, namely, a rack carrying mechanism, a rack recirculating line 4, analyzer modules 5, 6, 7 and 8, sample rack storage unit 9, a buffer 91, a sample rack recovering unit 10 and a main control computer 11.

The sample rack feed unit 1 feeds plural sample racks each holding plural assay samples. The analyzer modules 5, 6, 7 and 8 are arranged along the rack carrying line 3 and are detachably connected to the rack carrying line 3. The automated analyzer may be provided with any number of analyzer modules. The automated analyzer in this embodiment is provided with the four analyzer modules 5, 6, 7 and 8. Although the analyzer modules 5, 6, 7 and 8 of this embodiment are biochemical analyzer modules, the automated analyzer may be provided with gene analyzer modules and immunity analyzer modules in combination with the analyzer modules.

The rack carrying line 3 carries the sample rack fed by the sample rack feed unit 1 to specified ones of the analyzer modules 5, 6, 7 and 8. The rack carrying line 3 carries the sample racks holding assay samples analyzed by the analyzer modules 5, 6, 7 and 8 to the sample rack recovering unit 10. The analyzer modules 5, 6, 7 and 8 have siding lines 51, 61, 71 and 81, respectively.

The sample rack is moved from the carrying line 3 to the siding lines 51, 61, 71 and 81 in the analyzer modules 5, 6, 7 and 8 to deliver the sample rack to the analyzer modules 5, 6, 7 and 8. The sample rack holding an assay sample processed by an analytical process by some one of the analyzer modules 5, 6, 7 and 8 is carried along the rack recirculating line 4 to the entrance of the carrying line 3 when the assay sample needs to be subjected to the analytical process again or when the assay sample needs to be subjected to another analytical process.

A buffer 91 is placed in the carrying line 3 or the rack recirculating line 4. The sample rack holding an assay sample can be stored for an optional time in the buffer 91. The sample rack is delivered from the buffer 91 at a desired time to the analysis module or the sample rack recovering unit 10. A sample rack holding quality control materials and standard samples which are to be analyzed at specified times can be stored in the buffer 91 and can be delivered to the analyzer modules at specified times.

When each assay sample analyzed by each analyzer module is to be analyzed by another analyzer module, a sample rack storage unit 9 stores temporarily the sample rack holding the assay sample until a decision is made as to whether or not the assay sample is to be analyzed by another analyzer module after the analyzer module has completed dispensation and analysis.

The analyzer modules 5, 6, 7 and 8 are provided with analyzer module control computers 12, 13, 14 and 15, respectively. The analyzer module control computers 12, 13, 14 and 15 control processes to be carried out by the analyzer modules 5, 6, 7 and 8, respectively. The sample rack feed unit 1 is provided with a computer 16 for controlling operations of the sample rack feed unit 1, the carrying line 3, the rack recirculating line 4, the buffer 91 and the sample rack recovering unit 10.

The sample rack storage unit 9 is provided with a computer 17 for controlling operations relevant to the sample rack. The computers 12, 13, 14, 15, 16 and 17, and the ID reader unit 2 are connected to a main control computer 11. Connected also to the main control computer 11 are an operating unit 18 provided with an I/O device for entering necessary information, and a display unit 19 for displaying results of analysis.

Each of the assay samples held on the sample rack carries sample ID information about the assay sample including reception number, patient's name, specified analyses, etc. The sample rack carries rack ID information including rack number. The sample rack delivered to the sample rack feed unit 1 is carried by the carrying line 3. Upon the transference of the sample rack from the sample rack feed unit to the carrying line 3, the ID reader unit 2 reads the sample ID information and the rack ID information and sends the same to the main control computer 11. Then, the main control computer 11 selects the analyzer modules for carrying out the specified analyses on the basis of the sample ID information and gives the information to the analyzer control computers included in the chosen analyzer modules among the analyzer computers 12, 13, 14 and 15.

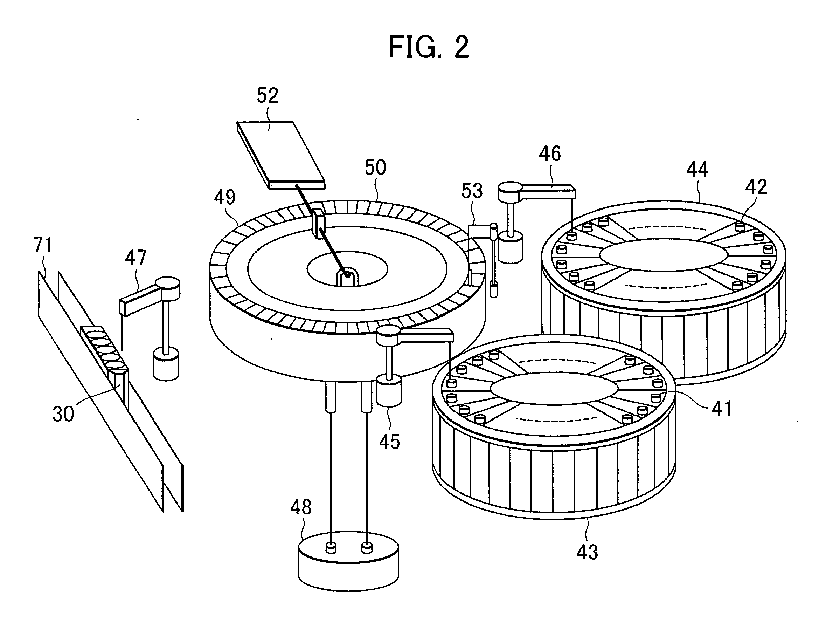

The constitution of the analyzer module 5 shown in Fig. 1, namely, biochemical analyzer module, for the automated analyzer embodying the present invention, namely, an analyzing system, will be described by way of example with reference to Fig. 2 showing the analyzer module 5 in a plan view.

Fig. 2 is a perspective view of a biochemical analyzer module included in the automated analyzer in this embodiment.

The analyzer module 5 is provided with a reagent system including a first reagent disk 43 provided with plural first reagent containers 41 circularly - arranged and containing a first reagent, a second reagent disk 44 provided with plural second reagent containers 42 circularly - arranged and containing a second reagent, a first reagent dispensing pipette device 45, and a second reagent dispensing pipette device 46. The analyzer module 5 is provided also with a sample system including a sample dispensing pipette device 47, a reaction system including a thermostatic bath, and a reaction disk 49, through which a constant-temperature fluid supplied from the thermostatic bath flows, provided with plural reaction vessels 50, a measuring system, namely, an assay system, including a multiple-wavelength photometer 52.

A sample rack 30 is moved into a siding line 71. The sample dispensing pipette device 47 sucks an assay sample contained in a sample container held on the sample rack 30 and positioned at a sample sucking position and pipettes the assay sample into the reaction vessel 50 of the reaction disk 49 positioned at a sample dispensing position. The reaction disk 49 is turned to position the reaction vessel 50 containing the assay sample at a first reagent dispensing position. Then, the first reagent pipette device 45 dispenses the first reagent contained in the first reagent container 41 held on the first reagent disk 43 to the reaction vessel 50.

Then, the reaction vessel 50 containing the assay sample and the first reagent is moved to a stirring position, where a stirring device 53 stirs the assay sample and the first reagent contained in the reaction vessel 50.

If a second reagent needs to be added to the mixture of the assay sample and the first reagent, the reaction vessel 50 is moved to a second reagent dispensing position, and then, the second reagent pipette device 46 dispenses the second reagent contained in the second reagent container 42 held on the second reagent disk 44 to the reaction vessel 50. Then, the reaction vessel 50 is moved to the stirring position and the stirring device 53 stirs the assay sample, the first reagent and the second reagent to produce a reaction mixture.

The reaction vessel 50 containing the reaction mixture is moved to a measuring position. Then, the multiple-wavelength photometer 52 measures the multiple-wavelength absorbance of the reaction mixture to determine biochemical values of the assay sample.

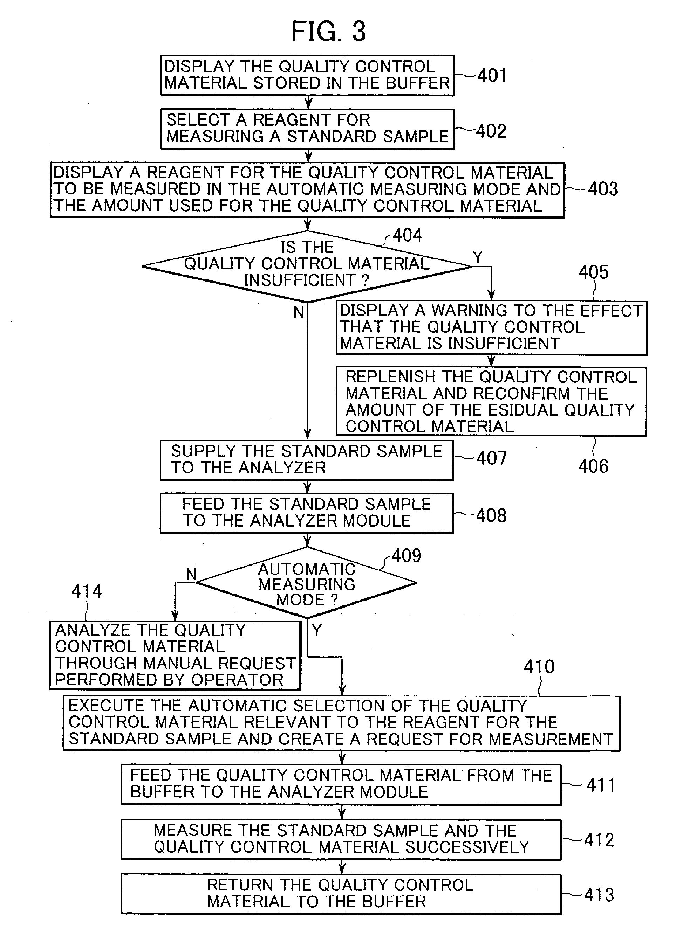

A quality control procedure that is carried out by the automated analyzer according to the embodiment will be described with reference to Fig. 3. When a standard sample is supplied to the automated analyzer, a quality control material relevant to the same reagent as a requested one is selected and quality control measurement is carried out. The following description will be made on an assumption that a requested standard sample relevant to the specified reagent used by the analyzer module 5 is supplied to the automated analyzer while the automated analyzer is in operation for carrying out an automated analyzing process. The same quality control procedure is executed by the other analyzer modules 6, 7 and 8 when a request for a standard sample is made or the plural analyzer modules make a request. The automated analyzer executes the same quality control procedure when the same is provided with a single analyzer module.

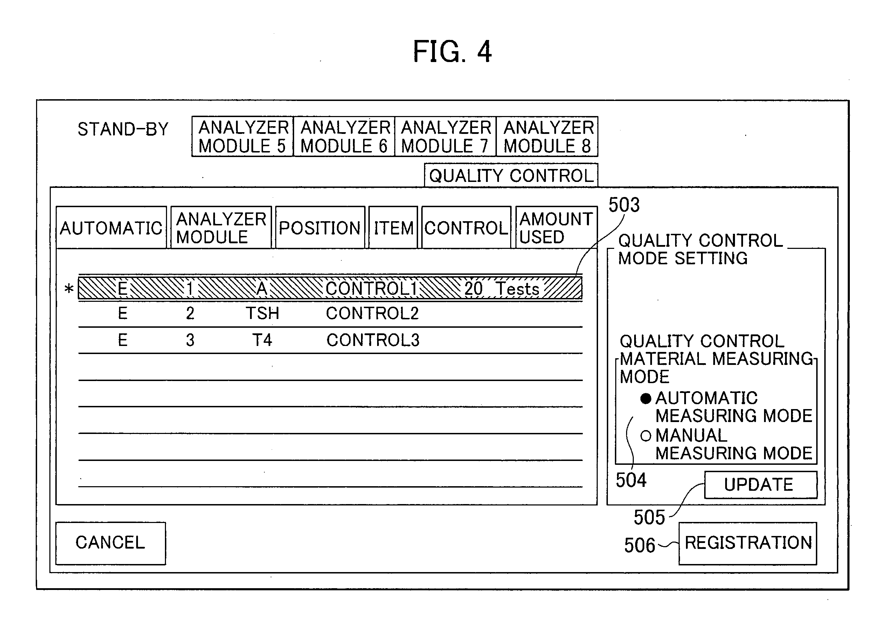

Information as shown in Fig. 4 about a quality control material stored in the buffer 91 is displayed in step 401. For example, in Fig. 4, indicated at 501 are reagents A, TSH and T4, and indicated at 502 are stored controls 1, 2 and 3 relevant to the reagent.

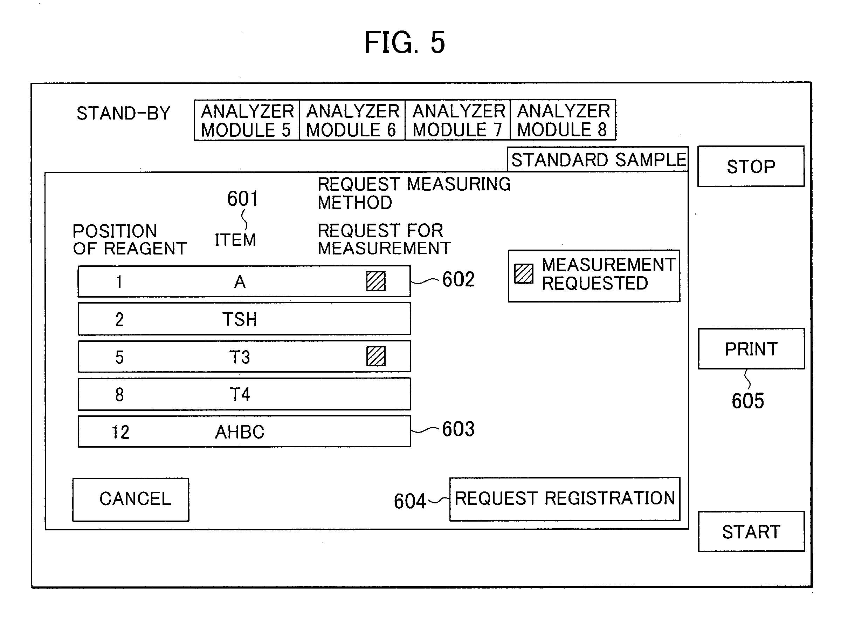

In step 402, "Request" is selected for an item indicated at 601 in Fig. 5 for requesting the measurement of the standard sample. When measurement is requested, a mark indicated at 602 is displayed and a mark indicated at 602 is displayed when measurement is not requested. "Request registration" indicated at 604 is depressed to complete setting selected items.

In step 403, either of an automatic quality control material measuring mode or a manual quality control material measuring mode shown in a radio box 504 is selected for the standard sample. The operation in the manual quality control material measuring mode is performed when the operator requests it at an optional time. If the automatic quality control material measuring mode is selected, the item name of the quality control material indicated at 501 to be tested by using the reagent for testing the standard sample requested and registered in step 402, the quality control material indicated at 502 and the amount used of the standard sample indicated at 503 are displayed. If the manual quality control material measuring mode is selected, manually selected items, the quality control material and the amount used of the quality control material are displayed. In Fig. 4, the automatic quality control material measuring mode is selected, the amount used of the quality control material is represented by the number of tests performed by way of example. In Fig. 4, the number of tests performed is twenty. When a print button indicated at 605 is depressed, the item, the quality control material and the amount used of the quality control material are printed by an output device.

In step 404, the main control computer 11 compares the amount used indicated at 503 of the quality control material indicated at 502 with the amount of the residual quality control material stored in the main control computer 11, and the main control computer 11 decides whether or not the amount of the residual quality control material is insufficient.

If it is decided in step 404 that the amount of the residual quality control material is insufficient, the color of the displayed amount used indicated at 503 is changed to warn the operator in step 405. The operator is prompted to replenish the quality control material in step 406. Thus the operator can know the amount of the residual quality control material and replenish the quality control material if the amount of the residual quality control material is insufficient.

If it is decided in step 404 that the amount of the residual quality control material is sufficient, the sample rack holding a container containing the standard sample is placed in the sample rack feed unit 1 in step 407.

In step 408, the sample rack holding the container containing the standard sample is delivered to the ID reader unit 2, the ID information is read to identify the standard sample, and the sample rack is carried to the analyzer module 5 which can measure the item A indicated at 602 indicating that the ready for measurement mark is given.

If the automatic quality control material measuring method is selected as the quality control material measuring method indicated at 504, step 410 is executed. In step 410, the main control computer 11 automatically selects the control 1 stored as a quality control material indicated at 502 relevant to the item A indicated by the request mark indicated at 602, and a request for the measurement of the item name A for the control 1 is produced. If plural controls 1 are stored as the quality control materials indicated at 502 in the buffer 91 when the control 1 is selected, the quality control material of the control 1 deemed sufficiency of the material from the amount used indicated at 503 and the number of the residual controls 1 is selected.

In step 411, a sample rack holding a container containing the quality control material indicated at 502 stored in the buffer 91 is fed to the analyzer module 5 subsequently to feeding the rack holding the standard sample in step 408.

In step 412, the biochemical values of the item name A of the standard sample and the quality control material supplied to the analyzer module 5 are measured.

After the completion of dispensation for the measurement, the rack holding the container containing the standard sample is carried to the sample rack recovering unit 10 and the sample rack holding the container containing the quality control material is returned to the buffer 91 in step 413 to prepare for the next request of measurement for quality control.

If the manual quality control material measuring mode indicated at 504 is selected in step 407, the operator can request the measurement of the quality control material using the reagent at optional time to perform an analysis.

## Claims

1. An automated analyzer provided with analyzer units for assaying biochemical samples, and a sample carrying device for carrying the biochemical samples to the analyzer unit, said automated analyzer comprising:
registering means for registering standard samples and quality control materials relevant to reagent beforehand; and
control means for receiving a request for analyzing the registered standard sample, and giving an instruction to execute automatically the analysis of a quality control material registered by the registering means in combination with the standard sample.

2. The automated analyzer according to claim 1, wherein the analyzer units are connected to the sample carrying device.

3. The automated analyzer according to claim 1 or 2, wherein the standard samples and the quality control materials are stored in a buffer capable of feeding a desired one of the standard samples or the quality control materials to the sample carrying device.

4. The automated analyzer according to claim 1 further comprising means for outputting, when an instruction to measure the quality control material is given by the control means, at least a name of a relevant reagent, a name of the specified quality control material or an amount of the quality control material to be used before starting the measurement of the quality control material.

5. The automated analyzer according to claim 4 further comprising:
storage means for storing amounts of the residual quality control materials stored in buffer; and
warning means for generating a warning signal before starting the measurement of the quality control material when the control means provides an instruction to measure the quality control material if the amount of the residual quality control material stored in the storage means is compared with the amount of the quality control material to be used so as to determine that the quality control material is insufficient.

6. The automated analyzer according to claim 5, further comprising control means capable of performing a control operation to select a sufficient quality control material among plural quality control materials registered by the registering means for the same reagent and to measure the selected quality control material.

7. The automated analyzer according to claim 1, further comprising registering means for registering an automatic measuring mode in which the measurement of the quality control material is requested automatically or a manual measuring mode in which the measurement of the quality control material is requested manually.
